# EUROPEAN PATENT APPLICATION

(11) **EP 4 647 495 A1**
(43) Date of publication of application: **12.11.2025**
(21) Application number: 24738520.6
(22) Date of filing: 04.01.2024
(51) Int. Cl.: C12N 15/113, C12N 15/85, C12N 5/10, C12P 21/00

(54) **TRUNCATED INSULATOR AND APPLICATION THEREOF**

(30) Priority: 05.01.2023 CN 202310012420
(71) Applicant: Shanghai Qilu Pharmaceutical Research and Development Centre Ltd., Shanghai 201203 (CN)
(72) Inventor: CHEN, Jie, Shanghai 201203 (CN); SHI, Lani, Shanghai 201203 (CN); JIN, Congcong, Shanghai 201203 (CN); WANG, Yan, Shanghai 201203 (CN); LI, Mingyuan, Shanghai 201203 (CN); QI, Jiali, Shanghai 201203 (CN); ZHANG, Kang, Shanghai 201203 (CN); CHEN, Xi, Shanghai 201203 (CN); FANG, Yan, Shanghai 201203 (CN); LIU, Jun, Shanghai 201203 (CN)
(74) Representative: Schmitz, Joseph
(86) International application number: PCT/CN2024/070516
(87) International publication number: WO 2024/146587

(57) **Abstract**

Provided are a truncated insulator and an expression system comprising the insulator and applied to a eukaryotic cell. The truncated insulator breaks through the setting of an original core area, shortens an effective sequence of the insulator, and is more beneficial to the integration of the insulator and other elements of a carrier. The insulator can significantly improve the yield of a protein and obtain a recombinant cell strain having good genetic stability in an expression system in which a transposon (such as PiggyBac) is present.

## Description

The present application claims the priority to a Chinese Patent Application No. 202310012420.0, filed with the China National Intellectual Property Administration (CNIPA) on January 5, 2023, titled "TRUNCATED INSULATOR AND APPLICATION THEREOF", which is incorporated herein by reference in its entirety.

### Technical Field

The application relates to the field of biotechnology, in particular to a truncated insulator and application thereof.

### Background

Currently, there are hundreds of approved antibody drugs worldwide, and antibody drugs in various therapeutic fields are increasingly benefiting patients. With the development of antibody drugs, the antibody market size has reached 100 billion US dollars, and the production scale can reach up to 10,000 liters per batch. In the antibody production process, cell strain construction, as the basis of antibody production, has been widely studied and concerned. How to achieve rapid research and development with high production of antibody drugs through cell strain construction has always been a goal pursued by the industry.

In the process of constructing stably transfected cell strains, the gene of interest is usually inserted into the host cell genome by random integration, which has the following defects: the integration efficiency is usually less than one in ten thousand, and the construction of stably transfected cell strains relies on a large amount of screening, which is labor-intensive and time-consuming; the gene of interest is easily affected by position effects and has a low expression level; the gene of interest is easily silenced by the host cell and has poor stability; the cell pool has a low yield, making it difficult to quickly obtain sufficient protein in the early stage to meet the needs of new drug research and development.

Therefore, some site-specific and semi-site-specific integration technologies came into being. For example, the PiggyBac transposon expression system consists of a donor plasmid and a helper plasmid or in vitro transcribed mRNA. The donor plasmid carries the transposase recognition sequences 5'-end inverted repeat sequence (5ITR) and 3'-end inverted repeat sequence (3ITR). The helper plasmid or in vitro transcribed mRNA encodes PiggyBac transposase (PB transposase), which can recognize 5ITR and 3ITR, and is capable of shearing or replicating in the original position, and then inserting into a specific position of the host genome with cyclization and the assistance of transposase to achieve a precise integration, which has significant advantages such as high integration efficiency, good stability, and uniform copy form, etc. However, at the same time, transposon integration technology has the defect of low expression level which results in high production costs and is difficult to be applied in large-scale production.

Studies have reported that insulator plays a vital role in regulating the spatiotemporal specific expression of eukaryotic genes. Insulator has the ability to protect gene of interests from the influence of surrounding regulators to avoid incorrect activation or silence of the gene, namely, has an enhancer blocking and heterochromatin barrier function, which can effectively inhibit the "position effect". HS4 is a DNase I hypersensitive site (HS) with an insulator function located upstream of the 5' end of the chicken β-globin locus, namely cHS4, which is about 1.2 kb in length. Studies have shown that it has both of the above functions. However, existing studies have shown that the insertion of the complete sequence will cause the vector to be too large, reduce its transfection efficiency, and thus affect the expression level of the protein. Therefore, it is necessary to conduct truncation research on cHS4. Studies have shown that the function of cHS4 depends on the 250-bp core region at the 5' end, but the function of this core region is incomplete and cannot play a good role in improving expression. The 400 bp region at the 3' end also plays an important role, and the function of cHS4-650 (sequence of 250-bp core region and 400-bp region at the 3' end) is better than that of cHS4-250. However, the 650-bp insulator still has the problem of causing the vector to be too large and reducing the transfection efficiency. Therefore, there is a technical need to further optimize the insulator sequence length and improve the transfection efficiency based on the 650-bp length.

There are also literature reports on methods of using the PiggyBac transposon expression system for early protein preparation, however, the results indicate that the protein expression level is relatively low and cannot meet the needs of industrial production. With the progress of transposon research, it is reported that adding truncated insulators to the flanks of the gene of interest can significantly increase the expression level of the transgene and reduce the coefficient of variation of gene expression, thereby improving stability. However, most of the current studies on the truncation of cHS4 insulator are based on the strategy of combining different segments that have been studied, mainly combining the 250-bp core region with other different segments, but fail to further optimize the length nor verify the function of the truncated insulator. None of the above studies was conducted on the construction of stably transfected cell strains for antibody drug production, and there is still a long way to go before industrial application.

### Summary of the Invention

The first aspect of the present application provides an insulator for expressing a polypeptide or protein in a eukaryotic cell. The nucleic acid sequence of the insulator comprises the nucleic acid sequence as set forth in SEQ ID NO. 1, SEQ ID NO. 2, SEQ ID NO. 3 or SEQ ID NO. 4, or comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98% or 99% identity with any one of the nucleic acid sequences as set forth in SEQ ID NO. 1, SEQ ID NO. 2, SEQ ID NO. 3 or SEQ ID NO. 4.

In some embodiments of the present application, the nucleic acid sequence of the insulator is as set forth in SEQ ID NO. 1, SEQ ID NO. 2, SEQ ID NO. 3 or SEQ ID NO. 4, or the nucleic acid sequence of the insulator is a nucleic acid sequence having at least 80%, 81%, 82%, 83%, 84%, 85%, 86%, 87%, 88%, 89%, 90%, 91%, 92%, 93%, 94%, 95%, 96%, 97%, 98% or 99% identity with any one of the nucleic acid sequences as set forth in SEQ ID NO. 1, SEQ ID NO. 2, SEQ ID NO. 3 or SEQ ID NO. 4.

The second aspect of the present application provides a eukaryotic cell expression system, which comprises the insulator as described in the first aspect of the present application, and the insulator can play a role in increasing protein production and cell strain stability.

In some embodiments of the present application, the eukaryotic cell expression system is a PiggyBac transposon expression system.

In some embodiments of the present application, the PiggyBac transposon expression system includes a PiggyBac transposon transgenic vector; preferably, the PiggyBac transposon expression system also comprises a PiggyBac helper vector or an mRNA encoding a transposase, wherein the PiggyBac helper vector contains a nucleic acid encoding the transposase.

In some embodiments of the present application, the PiggyBac transposon transgenic vector contains the insulator as described in the first aspect of the present application.

In some embodiments of the present application, the PiggyBac helper vector is pcDNA3.4.

In some embodiments of the present application, the PiggyBac transposon transgenic vector comprises a 5' end repeat sequence 5ITR and an inverted 3' end repeat sequence 3ITR, and the insulator is positioned between the 5ITR and the 3ITR; preferably, either two sequentially arranged insulators in forward and reverse complementary orientations, or two insulators both oriented in the forward orientation are positioned between the 5ITR and the 3ITR; preferably, a multiple cloning site is positioned between the insulators. Preferably, other elements of the PiggyBac transposon transgenic vector are derived from the original sequence of P3 vector.

In some embodiments of the present application, the PiggyBac transposon transgenic vector further comprises a selectable marker gene.

In some embodiments of the present application, the selectable marker gene is a glutamine synthetase gene (GS gene).

The third aspect of the present application further provides use of the insulator as described in the first aspect of the present application in the construction of a PiggyBac transposon transgenic vector or a PiggyBac transposon expression system.

The fourth aspect of the present application further provides use of the insulator as described in the first aspect of the present application or the eukaryotic cell expression system as described in the second aspect of the present application, in particular for the recombinant expression of a nucleic acid.

In some embodiments of the present application, the nucleic acid can encode a polypeptide or a protein, preferably an antibody, a fusion protein, an antigen or an enzyme. Preferably, the recombinant expression is performed in a eukaryotic host cell. Preferably, the expression system is a PiggyBac transposon expression system. Preferably, the eukaryotic host cell is a CHO cell.

The fifth aspect of the present application also provides use of the insulator as described in the first aspect of the present application or the eukaryotic cell expression system as described in the second aspect of the present application in constructing a recombinant cell strain.

In some embodiments of the present application, the host cell used by the recombinant cell strain is a mammalian cell, preferably a CHO cell.

In some embodiments of the present application, the construction of the recombinant cell strain comprises the following steps:
1) constructing a PiggyBac transposon transgenic vector containing a gene encoding the protein of interest and a glutamine synthetase gene, and a helper vector expressing the PB transposase or obtaining an mRNA encoding the PB transposase by in vitro transcription;
2) introducing the transposon transgenic vector and the helper vector, or the transposon transgenic vector and the mRNA encoding the PB transposase obtained in step 1) into a host cell by transfection;
3) placing the cells obtained in step 2) in a culture flask to recover for a period of time;
4) transferring the cells obtained in step 3) to a shake flask and screening them using GS;
5) performing an inoculation yield test after full recovery of the cell state to evaluate the antibody expression level.

In some embodiments of the present application, the protein of interest in step 1) may be an antibody, a fusion protein, an antigen or an enzyme or other types of protein or polypeptide.

In some embodiments of the present application, in step 1), an in vitro transcription method is used, and a DNA sequence encoding the PB transposase is used as a template, and the mRNA encoding the PB transposase is obtained by in vitro transcription, capping and tailing modification.

In some embodiments of the present application, in step 5), the full recovery of the cell state refers to a cell viability of not less than 95%.

In some embodiments of the present application, the vector construction in step 1) uses an endotoxin-free plasmid extraction kit.

In some embodiments of the present application, the transfection in step 2) is an electrotransfection.

In some embodiments of the present application, the culture flask in step 3) is a T bottle.

In some embodiments of the present application, a culture medium used in step 3) is CD CHO Fusion Medium + 6 mM glutamine, and the recovery time is 24 hours.

In some embodiments of the present application, a culture medium used in the shake flask and screening in step 4) is CD CHO Fusion Medium.

In some embodiments of the present application, the yield test in step 5) is a 7-day batch, the inoculation density is 5 ×10⁵ cells/mL, the culture medium is BM2, and the culture volume is 30 mL; 4 g/L of sugar is supplemented on the day 4 of culture, and the expression level in the supernatant is detected on the day 7 of culture.

The sixth aspect of the present application further provides a recombinant cell strain, wherein the recombinant cell strain comprises the insulator described in the first aspect of the present application or the eukaryotic cell expression system described in the second aspect of the present application; preferably, the host cell used by the recombinant cell strain is a mammalian cell, preferably a CHO cell.

Beneficial effects of the present application: The present application truncated the 250-bp core region of cHS4 for the first time, breaking the previous setting of the core region. Compared with the 650-bp cHS4 insulator sequence available in the prior art, the insulator sequence length is significantly shortened while the protein expression level is increased, the construction time of a stably transfected cell strain is shortened, and the constructed cell strain has a good genetic stability.

### Brief Description of the Drawings

The drawings described herein are used to provide further understanding of the present application and constitute a part of the present application. The exemplary embodiments of the present application and their descriptions are used to explain the present application and do not constitute improper limitations on the present application.
Fig. 1 is a schematic diagram of the structure of P3-AscI vector.
Fig. 2 is a schematic diagram of the structure of P3-5ITR-3ITR-cHS4 vector.
Fig. 3 is a schematic diagram of the structure of pcDNA3.4-PiggyBac vector.
Fig. 4 is a graph showing protein production within 6 days of transient transfection.
Fig. 5 is a graph showing protein production within 14 days of stable transfection.
Fig. 6A is a schematic diagram of the structure of P3-5ITR-R-3ITR-SEQ ID NO. 1 reverse complement cHS4 vector.
Fig. 6B is a schematic diagram of the structure of P3-5ITR-3ITR-SEQ ID NO. 1 forward cHS4 vector.
Fig. 7 is an effect diagram showing the clone growth stability of the PiggyBac transposon expression system.

### Detailed Description

In order to make the purpose, technical solutions, and advantages of the present application more clearly understood, the present application is further described in detail below with reference to the drawings and examples. Obviously, the described examples are only part of the examples of the present application, rather than all the examples. All other examples obtained by those skilled in the art based on the present application fall within the protection scope of the present application.

### Terms

All publications, patents, and patent applications mentioned in this specification are incorporated herein by reference to the same extent as if each individual publication, patent, or patent application was specifically and individually indicated to be incorporated herein by reference.

Before the present application is described in detail below, it is to be understood that this application is not limited to the particular methodology, protocols, and reagents described herein as these may vary. It is also to be understood that the terms used in the present application is for the purpose of describing specific embodiments only, and is not intended to limit the scope of the present application. Unless defined otherwise, all technical and scientific terms used herein have the same meaning as commonly understood by one of ordinary skill in the art of the present application.

Certain embodiments disclosed herein include numerical ranges, and certain aspects of the present application may be described in terms of ranges. Unless otherwise specified, it should be understood that the numerical range or the manner of describing in terms of ranges is only for the purpose of brevity and convenience, and should not be considered as a strict limitation on the scope of the present application. Therefore, the description using range format should be considered as specifically disclosing all the possible sub-ranges and all the possible specific numerical points within that range, as if such sub-ranges and numerical points had been expressly written in this application. The above principles apply equally regardless of the width or narrowness of the values stated. When a range is described, the range includes the endpoints of the range.

When referring to a measurable value such as an amount, a temporal duration, etc., the term "about" is meant to include variations of ±20%, or in some cases ± 10%, or in some cases ±5%, or in some cases ± 1%, or in some cases ±0.1% of the specified value.

The term "antibody" as used herein typically refers to a Y-shaped tetrameric protein comprising two heavy (H) polypeptide chains and two light (L) polypeptide chains held together by covalent disulfide bonds and non-covalent interactions. Natural IgG antibodies have such a structure. Each light chain consists of a variable domain (VL) and a constant domain (CL). Each heavy chain comprises a variable domain (VH) and a constant region.

As used herein, types of "antibody" in a broad sense may include polyclonal antibodies, monoclonal antibodies, chimeric antibodies, humanized antibodies and primatized antibodies, CDR-grafted antibodies, human antibodies (including human antibodies obtained by recombination), antibodies obtained by recombination, intracellular antibodies, multispecific antibodies, bifunctional fusion proteins, monovalent antibodies, multivalent antibodies, anti-idiotype antibodies, synthetic antibodies (including mutant proteins and variants thereof), and the like.

The term "PiggyBac transposon expression system" refers to the PiggyBac transposon vector system. The main members of the PiggyBac vector system comprise: a helper vector or plasmid, which encodes the transposase; a transposon vector (also called a donor vector) or plasmid, which contains optimized subterminal inverted terminal repeat sequences (ITR) at both ends and a region to be transposed in the middle, where the sequence of the gene of interest to be transposed into the host genome can be inserted. During the experiment, the helper plasmid and the transposon plasmid need to be transformed into the target cells at the same time. The transposase encoded by the helper plasmid recognizes the ITR sequences at both ends of the transposon plasmid and cuts them. The released region to be transposed is integrated, by the transposase, into the site containing TTAA sequence in the host genome, and TTAA repeat sequences appear at both ends of the region to be transposed. An mRNA transcribed in vitro encoding the transposase can also be used to replace the helper plasmid and to co-transform with the transposon plasmid into the target cells for the expression of the transposase.

The term "CHO platform" refers to the CHO cell strain screening technology platform. After CHO (Chinese hamster ovary cells) are adapted to the chemically defined medium CD CHO Fusion Medium, subclone screening is performed to establish a CHO cell line. Supporting reagents and processes such as the expression vector P3, culture medium for the clone construction stage, and fed-batch process platform culture medium, are also included.

The term "transgenic vector" refers to an expression vector that expresses a gene of interest. An expression vector refers to a vector that adds an expression element (such as promoter, RBS, terminator, etc.) to the basic skeleton of a cloning vector to enable the expression of the gene of interest. The gene of interest includes, but is not limited to, deoxynucleotide sequences encoding antibodies in a broad sense, antigens, fusion proteins, polypeptides and the like.

The term "insulator" or "insulator sequence" refers to a class of DNA sequences at the boundaries of chromatin domains. As a neutral barrier, it prevents the influence of adjacent genetic elements or surrounding compact chromatin, allowing the protected genes to be expressed in normal time and space. The effect of the insulator depends on the location thereof in the gene and the orientation of its own sequence. Exemplary insulator sequences include chicken hypersensitive site-4 (cHS4).

The term "multiple cloning site" refers to an artificially synthesized DNA fragment contained on a vector, which contains multiple single restriction enzyme cutting sites and is the insertion site of foreign DNA. It is also called a multi-site linker and is a standard configuration sequence of vector plasmids commonly used in genetic engineering. In a multiple cloning site, each restriction enzyme cutting site is usually unique, that is, they appear only once in a specific vector plasmid, and the enzyme cutting sites of different enzymes may overlap.

The term "transcription factor" (TF) refers to a group of protein molecules that can specifically bind to specific sequences upstream of the 5' end of a gene, thereby ensuring that the gene of interest is expressed with a specific intensity at a specific time and space.

The term "transcription factor binding site" (TFBS) refers to the region where a transcription factor binds to a gene template strand when regulating gene expression.

The term "Gene Ontology" (GO) is a standardized functional classification system that provides a dynamically updated standardized vocabulary and describes the properties of genes and gene products in organisms in three aspects: biological process (BP) involved, molecular function (MF), and cellular component (CC). GO term is the descriptive information of Gene Ontology function.

The term "Protein Protein Interactions" (PPI) refers to the use of protein interaction databases, such as the STRING protein interaction database, to directly extract the interaction relationships of the set the gene of interest (such as the differentially expressed gene list) from the database to construct a network for the species contained in the database.

The term "sequence identity" or "sequence similarity" or "sequence homology" refers to the percentage of nucleotides/amino acid residues in a candidate sequence that are identical to those in a reference sequence, after aligning the sequences (and introducing gaps, if necessary) to achieve the maximum percent sequence identity, and not considering any conservative substitutions as part of the sequence identity. Sequence alignment for determination of percent nucleotide/amino acid sequence identity can be achieved by various methods in the art, for example, using publicly available computer software such as BLAST, BLAST-2, ALIGN or MEGALIGN (DNASTAR) software. Those skilled in the art can determine appropriate parameters for measuring alignment, including any algorithms needed to achieve maximal alignment over the full length of the sequences being compared.

The term "recombinant expression" refers to an oligonucleotide or polynucleotide construct comprising a genetic modification, which allows a host cell to express an mRNA, protein, polypeptide or peptide when the construct is contacted with the host cell under conditions sufficient to allow expression of the mRNA, protein, polypeptide or peptide in the host cell, wherein the construct comprises a nucleotide sequence encoding the mRNA, protein, polypeptide or peptide.

The present application is further described in detail by the following examples. Specific examples are recited below to illustrate the present application. However, it should be understood that these examples are recited only to illustrate the present application rather than to limit the scope of the present application.

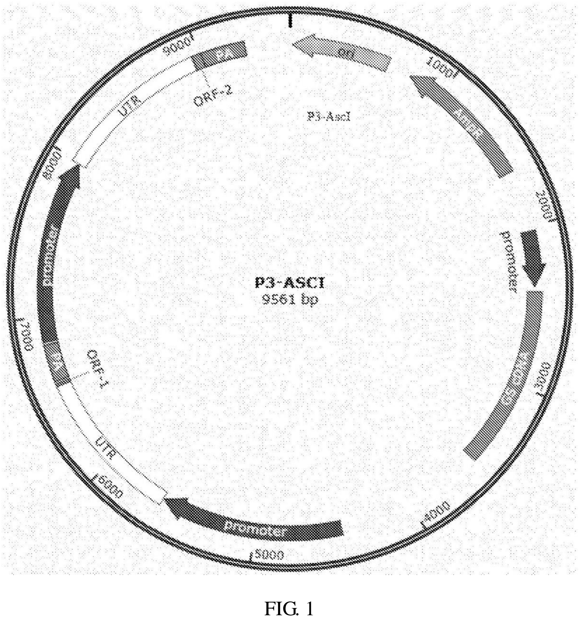

Materials and Reagents:
P3-AscI (schematic structure is shown in Fig. 1), having the AscI restriction site recognition sequence GGCGCGCC inserted into the P3 plasmid.

The sequence coding PiggyBac transposase was optimized for CHO cells by SYNBIO TECHNOLOGIES Co., Ltd., Suzhou (adding EcoRI restriction site and kozak sequence to the 5' end flank and HindIII to the 3' end flank) and subjected to gene synthesis, and cloned into the vector PUC57 to obtain PUC57-PiggyBac.

5ITR-cHS4 sequence in the PiggyBac donor vector element was synthesized by SYNBIO TECHNOLOGIES Co., Ltd., Suzhou.

3ITR-cHS4 sequence in the PiggyBac donor vector element was synthesized by GENEWIZ Biotechnology Co., Ltd, Suzhou.

3ITR-R-cHS4 sequence containing the reverse complementary insulator in the PiggyBac donor vector element was synthesized by GENEWIZ TECHNOLOGIES Co., Ltd, Suzhou, and is cloned into the PUC57 vector to obtain PUC57-3ITR-R-cHS4.

The template DNA sequence of the PiggyBac mRNA was synthesized by GenScript Biotech Co., Ltd, Nanjing.

The blank expression vector pcDNA3.4 was provided by GenScript Biotech Co., Ltd, Nanjing.

### Example 1: Design of cHS4 insulator truncated sequence and vector construction

### 1. Design and construction of truncated cHS4 insulator

In the insulators used, cHS4-650, cHS4-400, and cHS4-250 are obtained from publication (such as CN102943092A, WO2018083274A1, and US20150315611A1). The cHS4-650 sequence (including the 250-bp core region and the 400-bp sequence at the 3' end) was input into the AnimalTFDB3.0 online prediction website to predict possible binding transcription factors. The results showed that there were 3897 TFBSs corresponding to 389 transcription factors. Then, the STRING database was used to perform enrichment analysis on the 389 transcription factors, and a total of 1993 GO terms were significantly enriched. 91 transcription factors corresponding to 85 GO terms related to chromatin structure regulation or insulator function were selected for PPI analysis. Then, transcription factors with high connectivity and three transcription factors, CTCF, USF1/2, and VEZF1, which were reported to bind to the core region, were selected. According to the binding sequences predicted by AnimalTFDB3.0, these transcription factors were located on the cHS4-650 sequence. DNA regions with less or no binding by transcription factors were selected for truncation design, and single or multiple truncatable regions were combined for truncation. Finally, four insulators were obtained, and the nucleic acid sequences are as set forth in SEQ ID NO. 1, SEQ ID NO. 2, SEQ ID NO. 3 and SEQ ID NO. 4, specifically as follows: Take in the emidna (review instructions)
SEQ ID NO. 1 
cggggacagcccccccccaaagcccccagggatgtaattacgtccctcccccgctagggggcagcagcgagccgcccggggctccg
ctccggtccggcgctccccccgcatccccgagccggcagcgtgcggggacagcccgggcacggggaaggtggcacgggatcgctttcctct
gaacgcttctcgctgctctttgagcctgcagacacctggggggatacggggaaaatgtgtctgagcctgcatgtttgatggtgtctggatgcaagc
agaaggggtggaagagcttgcctggagagatacagctgggtcagtaggactgggacaggcagctatccccaagaccaaccccaacccaccca
ccgtgcccactggccatgtccttcatcacctccagggacggtgacccccccacctccgtgggcagctgtgccactgcagcaccgctctttggaga
aggtaaatcttgctaaatccagcccgaccctcccctggcacaacgtaaggccattatctct
SEQ ID NO. 2 
cggggacagcccccccccaaagcccccagggatgtaattacgtccctcccccgctagggggcagcagcgagccgcccggggctccg
ctccggtccggcgctccccccgcatccccgagccggcagcgtgcggggacagcccgggcacggggaaggtggcacgggatcgagcctgca
gacacctggggggatacggggaaaatgtgtctgagcctgcatgtttgatggtgtctggatgcaagcagaaggggtggaagagcttgcctggaga
gatacagctgggtcagtaggactgggacaggcagctatccccaagaccaaccccaacccacccaccgtgcccactggccatgtccttcatcacc
tccagggacggtgacccccccacctccgtgggcagctgtgccactgcagcaccgctctttggagaaggtaaatcttgctaaatccagcccgacc
ctcccctggcacaacgtaaggccattatctct
SEQ ID NO. 3 
cggggacagcccccccccaaagcccccagggatgtaattacgtccctcccccgctagggggcagcagcgagccgcccggggctccg
ctccggtccggcgctccccccgcatccccgagccggcagcgtgcggggacagcccgggcacggggaaggtggcacgggatcgctttcctct
gaacgcttctcgctgctctttgagcctgcagacacctggggggatgatgcaagcagaaggggtggaagagcttgcctggagagatacagctgg
gtcagtaggactgggacaggcagctatccccaagaccaaccccaacccacccaccgtgcccactggccatgtccttcatcacctccagggacg
gtgacccccccacctccgtgggcagctgtgccactgcagcaccgctctttggagaaggtaaatcttgctaaatccagcccgaccctcccctggca
caacgtaaggccattatctct
SEQ ID NO. 4

cggggacagcccccccccaaagcccccagggatgtaattacgtccctcccccgctagggggcagcagcgagccgcccggggctccg
ctccggtccggcgctccccccgcatccccgagccggcagcgtgcggggacagcccgggcacggggaaggtggcacgggatcgagcctgca
gacacctggggggatgatgcaagcagaaggggtggaagagcttgcctggagagatacagctgggtcagtaggactgggacaggcagctatcc
ccaagaccaaccccaacccacccaccgtgcccactggccatgtccttcatcacctccagggacggtgacccccccacctccgtgggcagctgtg
ccactgcagcaccgctctttggagaaggtaaatcttgctaaatccagcccgaccctcccctggcacaacgtaaggccattatctct

### 2. Donor vector construction

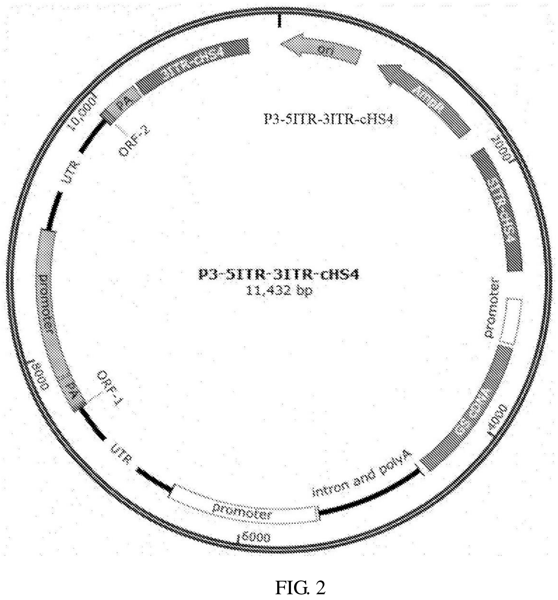

The PiggyBac donor vector element contains 5ITR-cHS4 (forward) and 3ITR-cHS4 (forward), wherein there are two forward cHS4s between 5ITR and 3ITR, and the cHS4 sequence contains cHS4-650, cHS4-400, cHS4-250, the sequence as set forth in SEQ ID NO. 1, the sequence as set forth in SEQ ID NO. 2, the sequence as set forth in SEQ ID NO. 3 or the sequence as set forth in in SEQ ID NO. 4. The 5ITR-cHS4 fragment was connected to the P3-AscI plasmid by AscI single enzyme digestion and homologous recombination to construct the plasmid P3-5ITR-cHS4, and then the 3ITR-cHS4 was integrated into the P3-5ITR-cHS4 by homologous recombination to construct the donor vector P3-5ITR-cHS4-3ITR-cHS4, which is abbreviated as P3-5ITR-3ITR-cHS4 below for convenience of expression (the structural schematic diagram is shown in Fig. 2).

### 3. Expression vector construction

The vector P3-5ITR-3ITR-cHS4 was double-digested with BstBI/PacI and HindIII/XhoI, and nucleic acid sequences encoding the heavy chain and light chain of QL01 monoclonal antibody were connected to the corresponding multiple cloning sites, respectively, to construct the recombinant expression vector P3-5ITR-3ITR-cHS4-QL01.

### 4. Helper vector construction

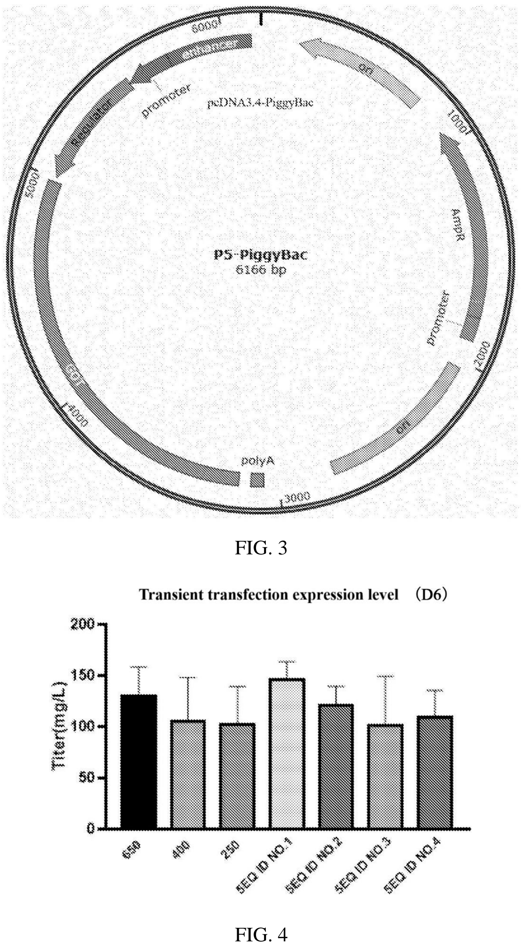

Vector PUC57-PiggyBac and blank vector pcDNA3.4 were double-digested with EcoRI/HindIII, and then the digested products of the vector PUC57-PiggyBac and the blank vector pcDNA3.4 were purified and recovered by NucleoSpin Gel and PCR Clean-up Kit, followed by ligation to construct a helper plasmid pcDNA3.4-PiggyBac (the structural schematic diagram is shown in Fig. 3) containing the gene encoding transposase. An in vitro transcription method can also be used, with the DNA sequence encoding PB transposase as a template, and the PB transposase-encoding mRNA-PiggyBac is obtained through in vitro transcription, capping and tailing modifications.

### Example 2: Transient transfection of cHS4 insulator truncated sequences for antibody expression

The host cell used was the ExpiCHO expression system (Gibco), and the transient expression of the protein was performed according to the recommended method for the host cell. The transient transfection system is 25 mL, and the amount of plasmid used for transfection is shown in Table 1. Feeding was performed according to the feeding scheme recommended by the ExpiCHO expression system kit, and culture was performed until the day 6. The antibody expression level was detected using the ForteBio instrument. The results are shown in Fig. 4. The culture medium was harvested after 7 days of culture.

Comparing the protein yields of ExpiCHO transient transfection, the yield of the PiggyBac transposon expression system with 650-bp (i.e., P3-5ITR-3ITR-650-QL01) cHS4 insulator was higher than that of the PiggyBac transposon systems with 400-bp (i.e., P3-5ITR-3ITR-400-QL01) and 250-bp (i.e., P3-5ITR-3ITR-250-QL01) insulators. Six days after transfection, the protein yield of the PiggyBac transposon expression system with the 650-bp cHS4 insulator was increased by 23% and 28% compared with the PiggyBac transposon expression systems with the 400-bp and 250-bp insulators, respectively. The above results show that adding 650-bp cHS4 insulator to the PiggyBac transposon expression system is most beneficial to improving the yield. Compared with 650-bp cHS4, the protein yield of the PiggyBac transposon expression system with the cHS4 insulator with the sequence as set forth in SEQ ID NO. 1 was increased by 13%, and the protein yield of the vectors P3-5ITR-3ITR-SEQ ID NO. 2-QL01 (containing the cHS4 insulator with the sequence as set forth in SEQ ID NO. 2), P3-5ITR-3ITR-SEQ ID NO. 3-QL01 (containing the cHS4 insulator with the sequence as set forth in SEQ ID NO. 3) and P3-5ITR-3ITR-SEQ ID NO. 4-QL01 (containing the cHS4 insulator with the sequence as set forth in SEQ ID NO. 4) after transfection was comparable to the that of the PiggyBac transposon expression system with the cHS4 insulator with the sequence as set forth in SEQ ID NO. 1.

**Table 1 Plasmids and amount of transient transfection**

| No. | Plasmid name | Amount of plasmid |
|---|---|---|
| 1 | P3-5ITR-3ITR-650-QL01 | 20 µg |
| 2 | P3-5ITR-3ITR-400-QL01 | 20 µg |
| 3 | P3-5ITR-3ITR-250-QL01 | 20 µg |
| 4 | P3-5ITR-3ITR-SEQ ID NO.1-QL01 | 20 µg |
| 5 | P3-5ITR-3ITR-SEQ ID NO.2-QL01 | 20 µg |
| 6 | P3-5ITR-3ITR-SEQ ID NO.3-QL01 | 20 µg |
| 7 | P3-5ITR-3ITR-SEQ ID NO.4-QL01 | 20 µg |

### Example 3: Stable transfection of cHS4 insulator truncated sequences for antibody expression

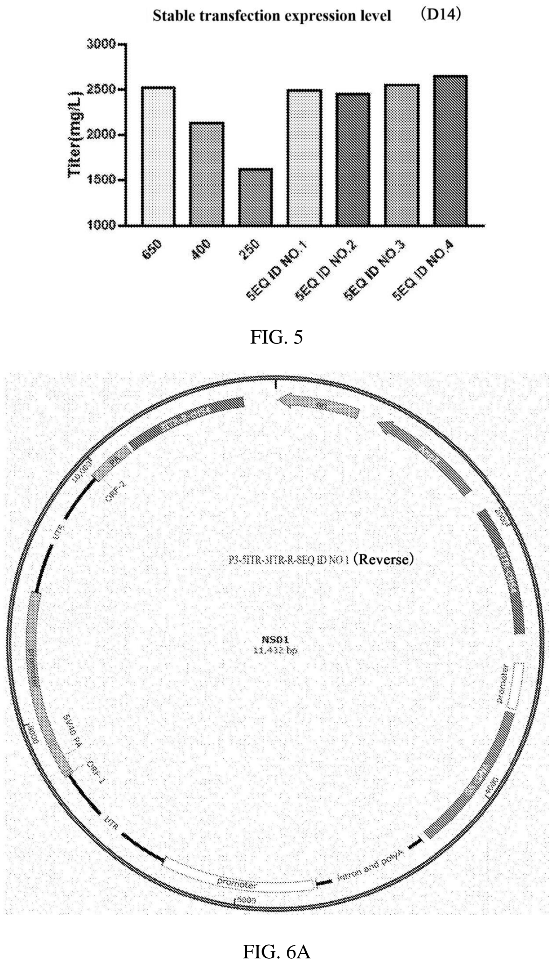

CHO cells were transfected by electrotransfection. The transfection conditions are shown in Table 2. After cell transfection, the cells were resuspended in the culture medium EX-CELL^{®} CD CHO Fusion Medium (containing 6 mM L-glutamine) and cultured in an incubator at 37 °C with 5% CO₂. After culturing for 24 hours, the cells were centrifuged at 1000 rpm for 5 min, the supernatant was removed and an appropriate amount of EX-CELL CD CHO Fusion culture medium was taken to resuspend the cells, and all were transferred to a 125 mL shake flask and cultured in a shaker at 37 °C, with 5% CO₂, 80% humidity, and 130 rpm. The cells were counted and passaged every 1-5 days until the viability of the cell pool was recovered. The cells were inoculated at a density of 5×10⁵ cells/mL for yield testing. The results of the fed batch on the day 14 of stable transfection expression are shown in Fig. 5. The results showed that the PiggyBac transposon expression systems containing insulators as set forth in SEQ ID NOs. 1-4 can all achieve good expression ability. In particular, the expression level of the PiggyBac transposon expression system comprising insulators as set forth in SEQ ID NO. 1, 3 or 4 is comparable to or higher than that of the PiggyBac transposon expression system comprising the 650 bp insulator, wherein the expression yield of the PiggyBac transposon expression system comprising a truncated sequence as set forth in SEQ ID NO. 4 increased by 5%, that is, the yield is increased by 134 mg/L. Compared with the insulator expression system comprising the core region of 250 bp, the expression yield of the PiggyBac transposon expression system comprising the truncated sequence as set forth in SEQ ID NO. 4 increased by 28%, that is, the yield is increased by 711 mg/L. Without being limited to any theory, the inventors believe that this may be because the truncated sequence not only retains the ability to optimize expression of the 650 bp insulator sequence used in the control group, but also improves the expression efficiency due to the shorter sequence and smaller vector. On the other hand, the PiggyBac transposon expression system comprising the insulators with sequences as set forth in SEQ ID NOs. 1-4 also has significant better expression ability than the PiggyBac transposon expression system comprising the insulators with the core region of 250 bp.

**Table 2 Conditions of stable transfection**

| No. | Plasmid | Amount of plasmid (µg) | Cell count | Transfection condition |
|---|---|---|---|---|
| 1 | P3-5ITR-3ITR-650-QL01 + pcDNA3.4-PiggyBac | 50+5 | 1.00E+07 | 300 V, 950 µF |
| 2 | P3-5ITR-3ITR-400-QL01 + pcDNA3.4-PiggyBac | 50+5 | 1.00E+07 | 300 V, 950 µF |
| 3 | P3-5ITR-3ITR-250-QL01 + pcDNA3.4-PiggyBac | 50+5 | 1.00E+07 | 300 V, 950 µF |
| 4 | P3-5ITR-3ITR-SEQ ID NO.1-QL01 + pcDNA3.4-PiggyBac | 50+5 | 1.00E+07 | 300 V, 950 µF |
| 5 | P3-5ITR-3ITR-SEQ ID NO.2-QL01 + pcDNA3.4-PiggyBac | 50+5 | 1.00E+07 | 300 V, 950 µF |
| 6 | P3-5ITR-3ITR-SEQ ID NO.3-QL01 + pcDNA3.4-PiggyBac | 50+5 | 1.00E+07 | 300 V, 950 µF |
| 7 | P3-5ITR-3ITR-SEQ ID NO.4-QL01 + pcDNA3.4-PiggyBac | 50+5 | 1.00E+07 | 300 V, 950 µF |

### Example 4: Effect of the orientation of the insulator in the transposon expression system on antibody expression

### 1. Construction of donor vector P3-5ITR-3ITR-R-SEQ ID NO. 1

Using the vector PUC57-3ITR-R-SEQ ID NO. 1 (containing a sequence that is reverse complementary to the sequence as set forth in SEQ ID NO. 1) as a template, PCR was performed to amplify the fragment of interest, 3ITR-R-SEQ ID NO. 1. The P3-5ITR-SEQ ID NO. 1 (forward) plasmid was single-digested with SgrAI, and the 3ITR-R-SEQ ID NO. 1 gene fragment was connected by homologous recombination to construct the vector P3-5ITR-SEQ ID NO. 1-3ITR-R-SEQ ID NO. 1, with a forward cHS4 and a reverse complementary cHS4 between 5TIR and 3ITR, i.e., P3-5ITR-3ITR-R-SEQ ID NO. 1 (the structural schematic diagram is shown in Fig. 6A).

### 2. Construction of vectors P3-5ITR-3ITR-SEQ ID NO. 1-QL02 and P3-5ITR-3ITR-R-SEQ ID NO. 1-QL02

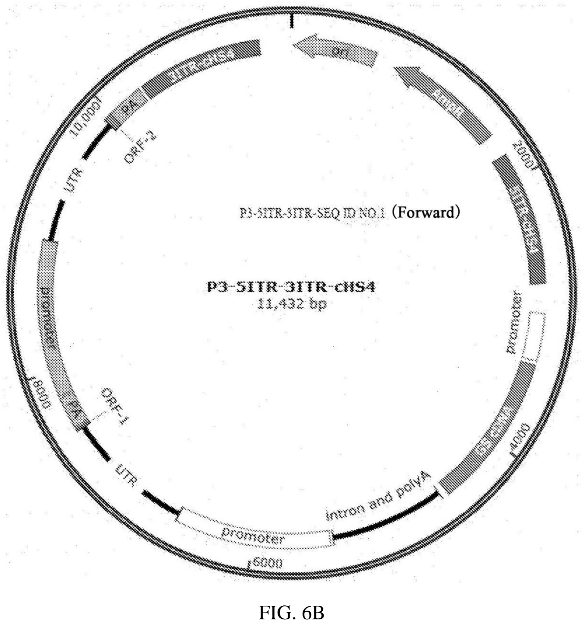

The nucleic acid sequences encoding Knob and Hole chains of QL02 bispecific antibody were connected to the corresponding multiple cloning sites of P3-5ITR-3ITR-SEQ ID NO. 1 (the structural schematic diagram is shown in Fig. 6B) and P3-5ITR-3ITR-R-SEQ ID NO. 1 by double restriction enzyme ligation using BstBI/PacI and HindIII/XhoI, respectively, to construct recombinant expression vectors P3-5ITR-3ITR-SEQ ID NO. 1-QL02 and P3-5ITR-3ITR-R-SEQ ID NO. 1-QL02.

### 3. Antibody protein expression

The host cell used was the ExpiCHO expression system (Gibco), and the transient expression of the protein was performed according to the recommended method for the host cell. The transient transfection system is 25 mL, and the amount of plasmid used for transfection is shown in Table 3. Feeding was performed according to the feeding scheme recommended by the ExpiCHO expression system kit, and culture was performed until the day 7. The antibody expression level was detected using the ForteBio instrument. The results are shown in Table 4.

**Table 3 Experimental groups of transient transfection**

| No. | Plasmid and amount |
|---|---|
| 1 | 25 µg P3-5ITR-3ITR-R-SEQ ID NO.1-QL02 +5 µg pcDNA3.4-PiggyBac |
| 2 | 25 µg P3-5ITR-3ITR-SEQ ID NO.1-QL02 +5 µg pcDNA3.4-PiggyBac |

**Table 4 Expression level on day 7 of transient transfection**

| No. | Plasmid name | Transient transfection expression level (mg/L) |
|---|---|---|
| 1 | P3-5ITR-3ITR-R-SEQ ID NO.1-QL02 | 102.1 |
| 2 | P3-5ITR-3ITR-SEQ ID NO.1-QL02 | 91.7 |

Comparing the expression quantities of the vector containing 3ITR+reverse complementary insulator (vector P3-5ITR-3ITR-R-cHS4) and the vector containing 3ITR + forward insulator (vector P3-5ITR-3ITR-cHS4) in the PiggyBac transposon expression system, the expression level of the vector containing 3ITR + reverse complementary insulator was 11.3% higher, indicating that the combination of vector 3ITR + reverse complementary insulator can more significantly improve the yield.

### Example 5: Use of stable transfection antibody expression of cHS4 insulator truncated sequence in the PiggyBac transposon system

### 1. Construction of donor vector P3-SITR-3ITR

The insulator in the vector P3-5ITR-3ITR-cHS4 was deleted to construct the P3-SITR-3ITR vector without an insulator.

### 2. Expression vector construction

The nucleic acid sequences encoding the light chain and heavy chain of antibody QL03 were respectively integrated into the light chain and heavy chain multiple cloning sites of P3-5ITR-3ITR-R-SEQ ID NO. 1 and P3-SITR-3ITR by HindIII/XhoI and BstBI/PacI enzyme ligation to construct expression vectors P3-5ITR-3ITR-R-SEQ ID NO. 1-QL03 and P3-5ITR-3ITR-QL03.

### 3. Transient transfection expression of antibody protein

The host cell used was the ExpiCHO expression system (Gibco), and the transient expression of the protein was performed according to the recommended method for the host cell. The transient transfection system was 50 mL, and the amount of plasmid used for transfection was 50 µg of donor vector + 5 µg of helper vector. Feeding was performed according to the feeding scheme recommended by the ExpiCHO expression system kit, and culture was performed until the day 7. The antibody expression level was detected using the ForteBio instrument. The results are shown in Table 5.

Comparing the yields 7 days after transient transfection, the yield of the PiggyBac transposon expression system with the cHS4 insulator was 247 mg/L, which increased by 1.5 times than that of the PiggyBac transposon expression system without the insulator. The above results indicate that adding cHS4 insulator to the PiggyBac transposon expression system can significantly increase the yield of cell strains.

**Table 5 Expression level of transient transfection**

| No. | Plasmid name | Transient transfection expression level (mg/L) |
|---|---|---|
| 1 | P3-5ITR-3ITR-QL03 | 89.2 |
| 2 | P3-5ITR-3ITR-R-SEQ ID NO.1-QL03 | 247.0 |

### 4. Stable transfection expression of antibody protein

CHO cells were transfected by electrotransfection. The amount of transfected plasmid was 50 µg of donor vector + 5 µg of helper vector, the voltage was 300 V, 950 µF, the cell number was 1E7, and the electric shock was performed once. After transfection, the cells were resuspended in the culture medium EX-CELL^{®} CD CHO Fusion Medium (containing 6 mM L-glutamine) and cultured in an incubator at 37 °C and 5% CO₂.

The cells were counted after culturing for 24 h to determine the viable cell density (VCD) and cell viability (Via), and minipools were plated. The cells were screened and expanded step by step in 96-well plates, 24-well plates and TPP. When the cells were fully recovered (viability>90%), they was inoculated at a density of 5 x10⁵ cells/mL for the Batch experiment to test yield. The yield results of the top 14 minipools are shown in Table 6.

Comparing the yield results of the stable transfection minipool yield test, the highest yield of the PiggyBac transposon expression system with the cHS4 insulator was 158 mg/L, which was 15.3% higher than the highest yield of the PiggyBac transposon expression system without the insulator. The average expression level of the top three yields was 150.6 mg/L, which was 54.9% higher than that without the insulator; and the average expression level was 112.3 mg/L, which was 102.4% higher than that without the insulator. The above results indicate that adding cHS4 insulator to the PiggyBac transposon expression system can increase the yield of cell strains.

**Table 6 Expression quantities of stable transfection minipool**

| Clone No. | Expression quantities of P3-5ITR-3ITR-QL03 (mg/L) | Expression quantities of P3-5ITR-3ITR-R-SEQ ID NO.1-QL03 (mg/L) |
|---|---|---|
| 1 | 137 | 158 |
| 2 | 83.1 | 154 |
| 3 | 71.8 | 140 |
| 4 | 71.6 | 120 |
| 5 | 67.1 | 111 |
| 6 | 59.8 | 106 |
| 7 | 49.4 | 106 |
| 8 | 48.9 | 106 |
| 9 | 45.8 | 101 |
| 10 | 40.7 | 98.5 |
| 11 | 38.2 | 96.3 |
| 12 | 32.6 | 95.4 |
| 13 | 19.8 | 92.9 |
| 14 | 10.6 | 86.1 |

### Example 6: Ensuring cell strain stability by PiggyBac transposon expression system

Three stably transfected cell strains constructed by the PiggyBac transposon expression system containing the insulator of SEQ ID NO. 1 were randomly selected. The cell strain construction method was as described in Example 5. The stability of the final monoclonal cells of the cell strain was evaluated, including: (1) growth stability: the cell strain was continuously cultured for 60 passages after thawing, and the cell density, viability, diameter and growth rate of the cells during the subculture process were evaluated; (2) production stability: the cells after subculture 30, 45 and 60 passages were tested for yield to evaluate the yield and quality of the product; (3) genetic stability: cell strains of the passages 0 and 60 were tested for cDNA sequencing.

### 1. Growth stability experiment

a) Candidate clones were thawed.
b) Every 2-3 days, the cells were passaged at a density of 2-5 × 10⁵ cells/mL, and the culture conditions were 37 °C, 80% humidity, 130 rpm, and 5% CO₂ concentration.
c) Cell density, viability and diameter during subculture were recorded and doubling time was calculated.

### 2. Production stability experiment

a) Cells were cryopreserved at PDL15, PDL30, PDL45, PDL60, PDL70.
b) After the subculture is completed, the cells of PDL15, PDL30, PDL45, PDL60, and PDL70 are thawed and inoculated for yield test after the cells are recovered.
c) Feeding was performed as needed during the culture process.
d) After the cell culture is completed, the cell culture supernatant is harvested, 1 mL of sample was taken and subjected to HPLC for testing, and the CV value was calculated according to the expression level: CV = (n PDL expression level - 0 PDL expression level) / 0 PDL expression level × 100%, n = 30/45/60.

### 3. Genetic stability experiment

After the stable subculture, the PDL0 and PDL60 cells were thawed, the total RNA was extracted and reverse transcribed into cDNA, and the gene of interest was amplified using the corresponding primers and then sequenced. The sequencing results were aligned with the theoretical sequence to confirm whether the DNA sequence of gene of interest mutated.

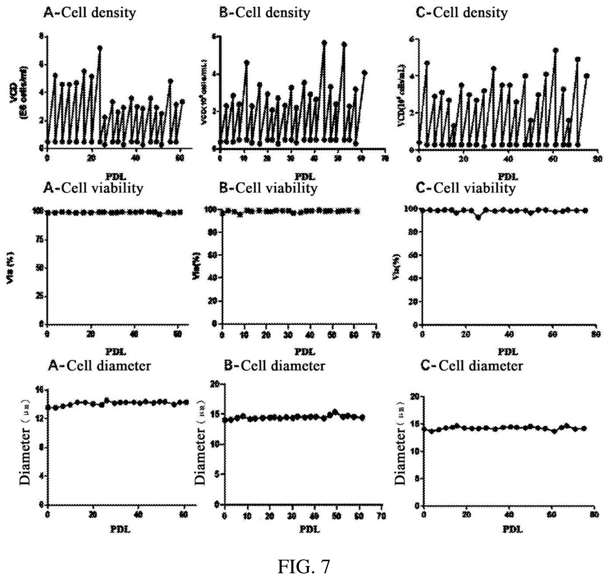

The growth stability data are shown in Fig. 7. After clones A, B, and C were passaged at the same density and cultured for the same time, there was no significant difference in cell density, the cell viability was maintained above 95%, and the diameter variation range was within 8%, indicating that the clones constructed by the PiggyBac transposon expression system remained stable in growth performance and cell morphology.

The cDNA sequencing results of clones A, B, and C were consistent with the theoretical sequences and showed no mutations. This indicates that the genetic characteristics of clones constructed by the PiggyBac transposon expression system did not change, and the gene of interest sequence is genetically stable after continuous cell passage. The changes in cell strain yields of the three projects were all within ± 10% (see Table 7 for specific data), and the range of change was much smaller than the industry average standard change of ± 30%, indicating that the system has a good genetic stability of cell strains.

**Table 7 Stability data of clone production constructed by PiggyBac transposon expression system**

| Clone name | Stable passage number | Yield (g/L) | CV% |
|---|---|---|---|
| A | P0 | 3.3 | / |
| | P30 | 3.5 | 7.0 |
| | P45 | 3.6 | 9.5 |
| | P60 | 3.6 | 8.2 |
| B | P0 | 4.5 | / |
| | P30 | 4.4 | -1.0 |
| | P45 | 4.2 | -5.9 |
| | P60 | 4.0 | -10.3 |
| C | P0 | 3.1 | / |
| | P30 | 3.3 | 10.2 |
| | P50 | 3.2 | 5.6 |
| | P60 | 3.3 | 6.1 |

The above are only preferred examples of the present application and are not intended to limit the present application. Any modifications, equivalent substitutions, improvements, etc. made within the spirit and principles of the present application shall be included in the scope of protection of the present application.

## Claims

1. An insulator, wherein the nucleic acid sequence of the insulator comprises a nucleic acid sequence having at least 80%, 81%, 82%, 83%, 84%, 85%, 86%, 87%, 88%, 89%, 90%, 91%, 92%, 93%, 94%, 95%, 96%, 97%, 98% or 99% identity with any one of the nucleic acid sequences as set forth in SEQ ID NO.1, SEQ ID NO.2, SEQ ID NO.3 or SEQ ID NO.4, or the nucleic acid sequence of the insulator comprises a nucleic acid sequence as set forth in SEQ ID NO.1, SEQ ID NO.2, SEQ ID NO.3 or SEQ ID NO.4.

2. The insulator according to claim 1, wherein the nucleic acid sequence of the insulator is a nucleic acid sequence having at least 80%, 81%, 82%, 83%, 84%, 85%, 86%, 87%, 88%, 89%, 90%, 91%, 92%, 93%, 94%, 95%, 96%, 97%, 98% or 99% identity with any one of the nucleic acid sequences as set forth in SEQ ID NO.1, SEQ ID NO.2, SEQ ID NO.3 or SEQ ID NO.4, or the nucleic acid sequence of the insulator is as set forth in SEQ ID NO.1, SEQ ID NO.2, SEQ ID NO.3 or SEQ ID NO.4.

3. A eukaryotic cell expression system, comprising the insulator according to claim 1 or 2.

4. The eukaryotic cell expression system according to claim 3, wherein the eukaryotic cell expression system is a PiggyBac transposon expression system.

5. The eukaryotic cell expression system according to claim 4, wherein the PiggyBac transposon expression system comprises a PiggyBac transposon transgenic vector; preferably, the PiggyBac transposon expression system further comprises a PiggyBac helper vector or an mRNA encoding a transposase, wherein the PiggyBac helper vector comprises a nucleic acid sequence encoding the transposase.

6. The eukaryotic cell expression system according to claim 5, wherein the PiggyBac transposon transgenic vector comprises a 5'-end repeat sequence 5ITR and an inverted 3'-end repeat sequence 3ITR, and the insulator is positioned between the 5ITR and the 3ITR; preferably, either two sequentially arranged insulators in forward and reverse complementary orientations, or two insulators both oriented in the forward orientation are positioned between the 5ITR and the 3ITR; and preferably, a multiple cloning site is positioned between the insulators.

7. The eukaryotic cell expression system according to claim 5 or 6, wherein the PiggyBac transposon transgenic vector comprises a selectable marker gene.

8. The eukaryotic cell expression system according to claim 7, wherein the selectable marker gene is a GS gene.

9. Use of the insulator according to claim 1 or 2 or the eukaryotic cell expression system according to any one of claims 3 to 8 in the recombinant expression of a nucleic acid.

10. The use according to claim 9, wherein the nucleic acid is capable of encoding an antibody, a fusion protein, an antigen or an enzyme.

11. Use of the insulator according to claim 1 or 2 or the eukaryotic cell expression system according to any one of claims 3 to 8 in the manufacture of a protein or a polypeptide.

12. The use according to claim 11, wherein the protein or polypeptide comprises an antibody, a fusion protein, an antigen and an enzyme.

13. Use of the insulator according to claim 1 or 2 or the eukaryotic cell expression system according to any one of claims 3 to 8 in the construction of a recombinant cell strain.

14. The use according to claim 13, wherein the host cell used by the recombinant cell strain is a mammalian cell, preferably a CHO cell.

15. A recombinant cell strain, wherein the recombinant cell strain comprises the insulator according to claim 1 or 2 or the eukaryotic cell expression system according to any one of claims 3-8; preferably, the host cell used by the recombinant cell strain is a mammalian cell, preferably a CHO cell.
